Europäisches Patentamt

European Patent Office

Office européen des brevets

(19)

(11) Publication number: **0 119 019**

**B1**

(12) **EUROPEAN PATENT SPECIFICATION**

(45) Date of publication of patent specification: **26.10.88**

(21) Application number: **84300938.2**

(22) Date of filing: **14.02.84**

(51) Int. Cl.⁴: **G 01 S 7/52, G 01 S 7/62, G 01 S 15/89, G 10 K 11/34**

(54) Ultrasonic diagnosis system.

(30) Priority: **14.02.83 JP 23548/83**

(43) Date of publication of application:
**19.09.84 Bulletin 84/38**

(45) Publication of the grant of the patent:
**26.10.88 Bulletin 88/43**

(84) Designated Contracting States:
**DE FR GB NL**

(56) References cited:
**EP-A-0 008 197**
**EP-A-0 012 165**
**AU-B- 490 105**
**DE-A-3 127 146**
**US-A-4 074 564**
**US-A-4 105 018**
**US-A-4 234 937**
**US-A-4 305 296**

(73) Proprietor: **HITACHI, LTD.**
**6, Kanda Surugadai 4-chome Chiyoda-ku Tokyo 100 (JP)**

(72) Inventor: **Umemura, Shinichiro**
**2-32-C403, Koyasu-cho**
**Hachioji-shi Tokyo (JP)**
Inventor: **Katakura, Kageyoshi**
**1-25-32, Naka-cho Meguro-ku**
**Tokyo (JP)**
Inventor: **Ogawa, Toshio**
**916-5, Ina, Itsukaichi-machi**
**Nishitama-gum Tokyo (JP)**

(74) Representative: **Calderbank, Thomas Roger et al**
**MEWBURN ELLIS & CO. 2/3 Cursitor Street London EC4A 1BQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to an ultrasonic diagnosis system.

Many studies on tissue characterisation have been made in recent years in various countries and one critical problem in such studies is the measurement of acoustic velocity. The conventional methods of measuring the acoustic velocity include a method known as "sing-around" and ultrasonic computer tomography. Since they are transmission methods, however, there is an inevitable limit to the measurement of the acoustic velocity inside the body of a subject.

On the other hand, measuring methods using reflection are known and such methods include one that involves the radiation of ultrasonic beams from two directions to the same target so as to make use of the refraction at the interface between the living body and an acoustic coupling solution. However, this method involves problems due to the low position accuracy of the two probes necessary and requires complicated processing for calculation of the acoustic velocity.

The present invention seeks to overcome this problem by making the set acoustic velocity variable.

DE—A—3 127 146 discloses a method of correcting image errors in an ultrasonic representation by varying the acoustic velocity assumed for different parts of an object to be imaged. The method generally employs a measured time difference between transmitted and received signals and geometrically measured distances to obtain an approximate acoustic velocity which can be used to produce a sharp image. To obtain an optimised image, an iterative process with a variety of assumed acoustic speeds may be carried out.

EP—A—1 21 65 proposes an alternative scheme for image focusing and relies on the shape of a transmitted ultrasonic beam and of its reception pattern, recognising that an optimum resolution region exists at the transition zone between near and far fields of the reception pattern. The method disclosed uses electronic focusing to continuously position the region of maximum reception resolution coincident with the locations within the subject from which ultrasonic echoes are being instantaneously generated by receding incident energy. This effect is achieved by increasing the number of active elements in a receiving transducer grouping and using a time delay to gradually bring in more active elements. The location of the transition zone, and hence of the optimum resolution region is dependent on the number of transducer elements applied for receiving. There is no suggestion in this specification that the assumed acoustic speed should be varied.

By varying the set acoustic velocity, in the present invention, the reflection characteristic detected by the receiver of the diagnosis system is studied until a maximum response is obtained. The true acoustic velocity can then be calculated.

Means are provided for displaying the set acoustic velocity so that the variation of an ultrasonic image produced from the reflection characteristic may be investigated.

According to the present invention there is provided an ultrasonic diagnosis system comprising:

an array having a plurality of oscillation elements for transmitting and receiving ultrasonic waves;

means for driving said oscillation elements so that pulse-like ultrasonic waves are transmitted from said array to an object;

means for detecting signals received by said oscillation elements, said signals being indicative of waves reflected from said object;

focusing means for delaying received signals at different oscillation elements and for summing the delayed signals, said signals being delayed according to a range of delay times determined so that a received ultrasonic beam is focused to a focal point;

display means for displaying an image of said object by converting an output of said focusing means to a visual signal;

characterised in that said ultrasonic diagnosis system further comrises;

signal generating means for generating an adjustable signal representative of an assumed acoustic velocity for said object, and

indicating means for indicating said assumed velocity;

and in that said focusing means is arranged to control said delay times as a function of said adjustable signal, whereby an actual acoustic velocity of said object is obtained from the indication of said indicating means when said signal generating means is adjusted so that the displayed image of a reflector in the object is in focus.

It is desirable that the ultrasonic waves are transmitted from or received at three mutually independent sources. By investigating the reflection characteristic of such a system the sensitivity of detection may be improved.

The set acoustic velocity may be made continuously variable or may vary stepwise, so that a range of acoustic velocities may be investigated automatically.

The present invention makes it possible to measure the acoustic velocity at a specific part or a specific organ inside a living body.

Embodiments of the invention will now be described by way of example, with reference to the accompanying drawings, in which:

Figure 1 is a schematic view of an ultrasonic beam;

Figure 2 is a diagram showing the change of acoustic pressure on the centre axis of the beam;

Figure 3 is a schematic view for explaining the delay time for focus;

Figure 4 is a schematic view of the space response function of a transmission-reception system;

Figure 5 is a diagram showing the construction of one variant of the present invention;

Figure 6 is a diagram showing the space response function of the transmission-reception system to a dot reflector in the system shown in Figure 5;

Figure 7 is a block diagram showing a first embodiment of the present invention;

Figure 8 is a diagram for explaining the operation of the circuit shown in Figure 7; and

Figures 9 to 21 are diagrams respectively showing other embodiments of the present invention.

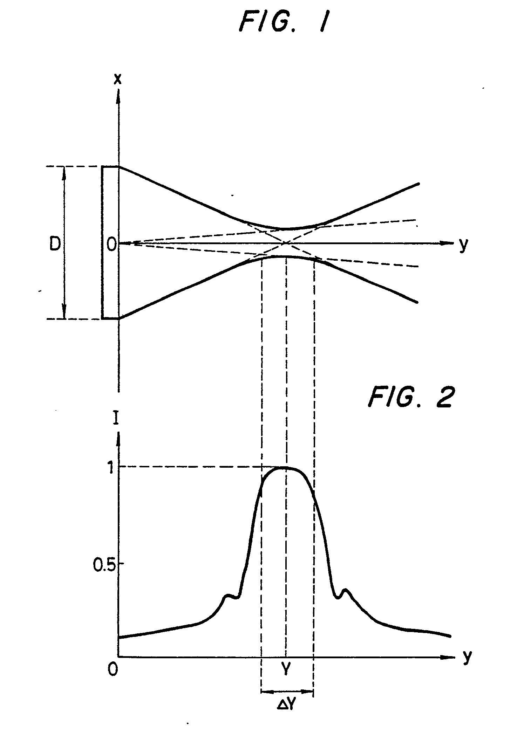

Figure 1 illustrates schematically the shape of a typical ultrasonic beam. In a transmission system, a physical beam is formed. In a reception system there is no physical beam, but a space response function can be obtained by consideration of a beam equivalent to a physical beam. In Figure 1, the diameter of transmission or reception is shown at D, and the x and y axes represent the direction of diameter and the direction of depth respectively.

Figure 2 illustrates the change of acoustic pressure I on the centre axis of the ultrasonic beam shown in Figure 1 as a function of the depth y.

The depth of focus $\Delta Y$ in the drawing can be expressed by the following equation

$$\Delta Y = 2 \pm Y^2/D^2 \qquad (1)$$

where Y is a focal length and $\lambda$ is the wavelength of the ultrasonic wave.

If a conventional ultrasonic system is to be operated easily the depth of focus $\Delta Y$ needed in the conventional ultrasonic diagnosis system to form a focus system having a fixed focus or a plurality of mutually spaced-apart focal points. However, this system is not convenient for measuring the acoustic velocity. In an ultrasonic diagnosis system according to the present invention, on the other hand, the depth of focus $\Delta Y$ should desirably be small in order to improve the measuring accuracy of the acoustic velocity.

Next, the measurement of the acoustic velocity achievable by the present invention will be explained in detail.

Assuming the acoustic velocity is $C_o$ and the set focal length is $Y_o$, then:

$$Y_o = C_o T_o \qquad (2)$$

where $T_o$ is half of the time required for a reflection signal to return.

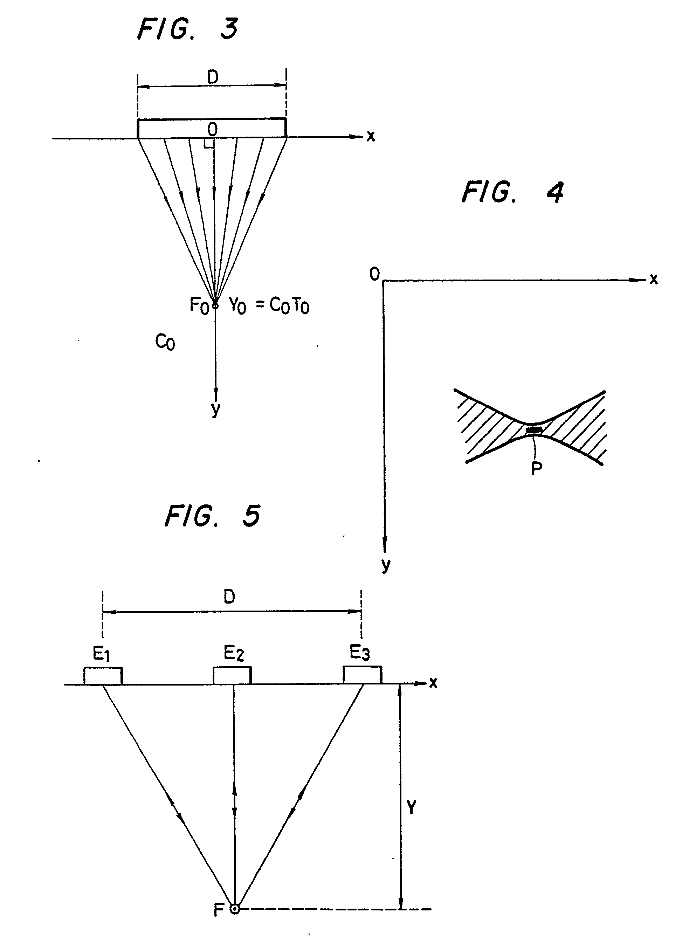

To focus the waves at the focal point, a pulse-like transmitting signal applied to an element positioned at coordinate x in Fig. 3 or a receiving signal from the element should be delayed by $\tau(x)$ as given by the following formula:

$$\tau(x) = \tau_o - (\sqrt{x^2 + Y_o^2} - Y_o)/C_o$$

$$\fallingdotseq \tau_o - x^2/(2C_o Y_o) \qquad (3)$$

where $\tau_o$ is a constant which is added so that

$\tau(x) \geqq 0$ for all x. When transmission and reception is effected in this way, the actual acoustic velocity C inside the body of the subject and the actual focal length Y are related by the following equation, with 2T (for x=0) representing the time required for the pulse actually to go to and return from the reflector at which the highest intensity of the reflection signal is obtained:

$$Y = CT \qquad (4)$$

$$\tau(x) \fallingdotseq \tau_o - x^2/(2CY) \qquad (5)$$

Therefore, from equation (3) and (5),

$$Y = C_o Y_o/C \qquad (6)$$

From equation (4) and (6):

$$C = \sqrt{\frac{C_o Y_o}{T}} \qquad (7)$$

As expressed by equation (7), it is possible in principle to obtain the actual acoustic velocity C from the assumed acoustic velocity $C_o$, the set focal length $Y_o$ and the time T required for a strong reflection signal to return.

The accuracy of acoustic velocity measurement $\Delta C/C_o$ is estimated from the following formula:

$$\frac{\Delta C}{C_o} \fallingdotseq \frac{\Delta T}{2T_o} = \frac{\Delta Y}{2Y_o} = \frac{\lambda Y}{D^2}$$

When $\lambda = 0.3$ mm, Y=100 mm and D=60 mm, for example, an accuracy of 0.8% can be obtained.

The above describes the fundamental principle of the present invention, but in practice it is difficult to measure the acoustic velocity inside the body of a subject by use of a conventional transmission-reception system having a fixed focus or a plurality of mutually spaced-apart focal points. This is because there is not necessarily a reflector having a sufficient reflecting intensity in the proximity of the focal position inside the body of the subject (the position whose distance from the body surface is Y given by equation (4)).

As shown schematically in Figure 4, the space response function of the transmission-reception system with respect to a point reflector takes a shape like a butterfly with spread wings, on both sides of a peak P and there is only a very small different between the shapes of the reflection images when the point reflector is at the focal point itself and when it has deviated from that point. Therefore, it seems more reliable to determine the deviation of setting of the acoustic velocity from the intensity rather than from the shape.

In the present invention, therefore, the set acoustic velocity $C_o$ is made variable. The ultrasonic image formed, and the set acoustic velocity $C_o$, may be displayed simultaneously. It then becomes possible to observe the ultrasonic image of the reflector having a sufficient reflection intensity by making fine adjustment of the focal length Y inside the body of the subject given by equation (6) and to measure accurately the time required for a strong reflection signal to return. As can be understood from equation (6), fine adjustment of $C_o$ is equivalent to fine adjustment of $Y_o$ in order to make fine adjustment of Y but the present specification will primarily deal with the former case for the sake of convenience.

The operator of the system can measure the acoustic velocity in the following manner, for example. First, the assumed acoustic velocity $C_o$ and the focal length $Y_o$ are set and the image formed is then observed. Next, the operator pays special attention to a reflector which is closed to the focal point but has a sufficient reflection intensity and adjusts the acoustic velocity $C_o$ so that the image of the reflector has the maximum intensity. The means acoustic velocity, which seems most reliable, from the body surface to the reflector can be obtained from $C_o$, $Y_o$ and T after completion of fine adjustment in accordance with equation (7).

In observing the image in the manner described above, it is not always easy accurately to determine the point where the image intensity is maximum. As shown in Figure 2, the acoustic pressure on the center axis changes only slightly within the depth of focus $\Delta Y$ described earlier so that the deviation of setting of the focal length or acoustic velocity cannot be determined easily with a high level of accuracy. The depth of focus $\Delta Y$ is inversely proportional to the square of the diameter D of transmission and reception as expressed by equation (1). For this reason, it is possible, in principle, to increase the diameter D in order to improve the sensitivity to the deviation of the set acoustic velocity by reducing $\Delta Y$. If this is to be achieved, however, the number of divisions of oscillation element must be increased, raising the cost of production of the system.

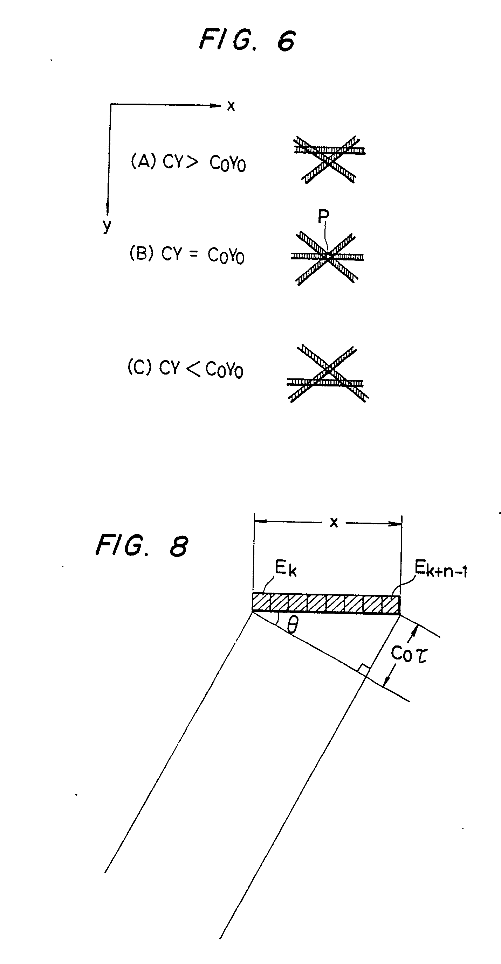

Therefore, it is a preferred feature of the present invention that the ultrasonic waves are transmitted or received from three mutually independent directions to the focus F as shown in Figure 5. The space-response function of the transmission-reception system to the point reflector in accordance with this system has a shape in which three straight lines cross one another. The great advantage of this system is that the shapes of the points of intersection of these lines undergo distinct changes in dependence upon the difference between the product $C_oY_o$ of the set acoustic velocity $C_o$ and the set focal length $Y_o$ and the product CY of the actual acoustic velocity C and the actual focal length Y, as depicted in Figures 6(A), (B) and (C). In this system, too, the diameter D must be increased as described above in order to improve the sensitivity to deviation of the set acoustic velocity but another advantage is that the number of channels of the oscillation elements to be used need not be increased.

The present invention also proposes a system for effecting so-called "real time dynamic focus reception" by continuously scanning and receiving the focal length by one transmission instead of effecting fine adjustment of the focus in order to measure the time T required for the strong reflection signal to return. This system continuously scans and receives $Y_o$ in equation (3) so as to satisfy the following formula, with t representing the time from the time of transmission:

$$Y_o = C_o t/2 \qquad (8)$$

The actual focal length inside the body of the subject is scanned as expressed by the following equation, by putting equation (8) into (6):

$$Y = (C_o^2/C) \times t/2 \qquad (9)$$

In a real time dynamic focus reception system, the clearest image can be obtained when Y is scanned in such a manner as to satisfy the relation $Y = C \times t/2$. As can be seen from equation (9), therefore, the clearest image or the highest image intensity can be obtained when $C_o$ coincides with C.

The operator of the system can measure the acoustic velocity in the following manner, for example. First, the assumed acoustic velocity $C_o$ is set. Next, the operator pays specific attention to a reflector having a sufficient reflector intensity and adjusts the acoustic velocity $C_o$ so that the image of the reflector has the maximum intensity. The $C_o$ value at the time of completion of adjustment represents the mean acoustic velocity itself, that seems most reliable, from the body surface to the reflector. In such a system, the operator can measure the acoustic velocity without considering $Y_o$ and T.

Figure 7 is a block circuit diagram of the system corresponding to the arrangement in which the ultrasonic waves are transmitted or received in three mutually independent directions. In the drawing, oscillation elements forming a split oscillator, are shown at E1 to EN, and elements selected by an element selecting circuit EC are shown at Ek to Ek+n−1. Also shown is a beam steering circuit B for transmission and reception, a frame memory M, an address signal generation circuit A for the frame memory M, a display circuit D and a velocity indicator V.

In the circuit construction described above, an echo signal obtained by transmitting and receiving the ultrasonic waves from three mutually independent directions is added in the frame memory.

Tha beam steering circuit B sends a transmission signal to the element selecting circuit EC in accordance with a transmission timing pulse $S_1$ and the address signal generation circuit A resets the y coordinates of the address. The element selecting circuit EC scans the selection element in accordance with a raster change-over signal $S_2$

and the address signal generation circuit A scans the x coordinates of the address. The beam steering circuit B also changes over the steering direction of transmission and reception in accordance with a steering change-over signal $S_3$ and the address signal generation circuit A changes over the address scanning direction. The reception signal sent from the beam steering circuit B to the frame memory M is written while containing phase data. Whenever scanning for one frame is completed, the data on the frame memory M are sent to the display circuit D and are simultaneously reset. The address signal generation circuit A changes, by only a limited amount, the address scanning direction in accordance with an assumed acoustic velocity signal 4. In other words, a shown in Figure 8, the address scanning angle $\theta$ is controlled in accordance with the assumed acoustic velocity $C_o$ so as to satisfy the relation:

$$\sin \theta = C_o \tau / x \qquad (10)$$

where x is the diameter used simultaneously and $\tau$ is the delay time difference between the two ends of the diameter.

In the system of this embodiment, there is no specific focal length and the reflection signal assumes the state shown in Figure 6(B) for the reflectors at all the distances when the acoustic velocity is in agreement with $C_o$. Thus, the operator observes the image and simultaneously adjusts the assumed acoustic velocity $C_o$. When the image of the reflector which is to be specifically observed reaches the state shown in Figure 6(B), the approximate value of C, which seems most reliable, may be obtained from the assumed acoustic velocity $C_o$ displayed by the velocity indicator V at that time.

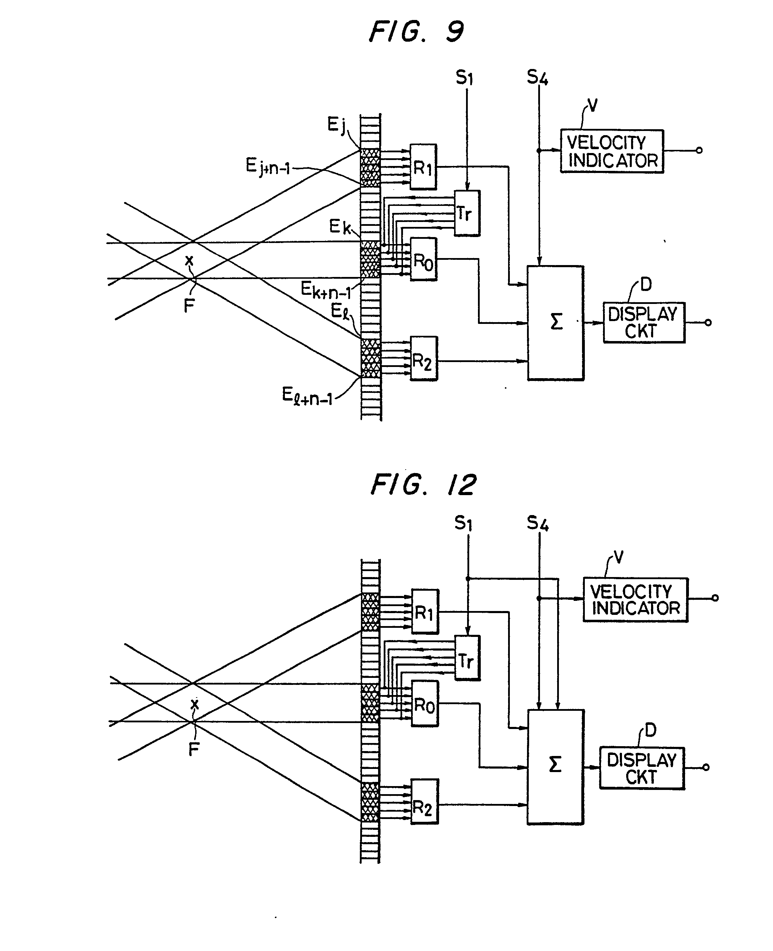

Figure 9 is a block circuit diagram showing a second embodiment of the present invention, which transmits a pulse-like ultrasonic wave signal from one oscillator block and receives the signal at three oscillator blocks formed by separating the reflection echo signal.

The oscillator blocks $E_K$ to $E_{K+n-1}$ forming the split oscillator transmit a pulse-like ultrasonic wave signal and the three oscillator blocks $E_j$ to $E_{j+n-1}$, $E_k$ to $E_{k+n-1}$ and $E_l$ to $E_{l+n-1}$ receive the signal. Also shown are the transmission pulse signal $S_1$ described above, a transmission circuit $T_r$ and reception circuits $R_o$, $R_1$ and $R_2$. The focus of reception is shown at F and a summation circuit $\Sigma$ for focusing can change the delay time by very small amounts in accordance with the assumed acoustic velocity.

In comparison with the system of the first embodiment shown in Figure 7, the system of the second embodiment has the advantage that errors due to the movement of the subject do not occur because the signals in the three directions necessary for photography are received simultaneously.

Incidentally, in the first embodiment shown in Figure 7, the relation between transmission and reception may be reversed and in such a case, only one set of reception circuits is used in place of the simultaneous use of three sets of reception circuits so that the cost of the transmission circuit may become more economical than the reception circuit.

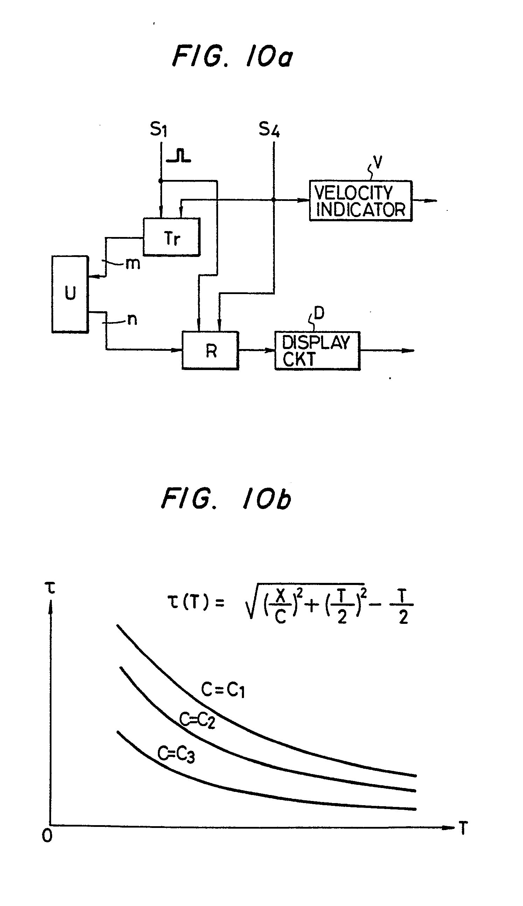

Figure 10a is a block circuit diagram of the system in accordance with a third embodiment. In the systems of this embodiment, the diameter of reception is set to be at least twice the diameter of transmission and photography is carried out while a large number of focal points are changed over for one transmission to take a photograph, thereby enabling a "real time dynamic focus reception method" to be achieved.

In the circuit of the third embodiment shown in Figure 10(a), the transmission timing pulse $S_1$ is applied to the transmission circuit $T_r$ and to the reception circuit R. The transmission circuit $T_r$ generates a transmission wave signal in synchronism with the transmission timing pulse $S_1$ in accordance with the value of the assumed acoustic velocity signal $S_4$ and sends it to an ultrasonic transducer array U. The reception circuit R effects dynamic focus reception while changing the focal length $Y_o$ in response to the time T from the transmission timing pulse $S_1$ in accordance with the value of the assumed acoustic velocity signal $S_4$ and sends the resulting image signal to the image display circuit D. The assumed acoustic velocity signal is also sent to the velocity indicator V.

Figure 10(b) shows the delay time $\tau$ given to the signal from one element in the reception diameter. To achieve dynamic focusing, $\tau$ is changed with T in accordance with the function shown in the Figure, but the function itself changes as shown in the Figure with the assumed acoustic velocity C as a parameter. Here, symbol x represents the distance between the element to be observed and the centre of diameter.

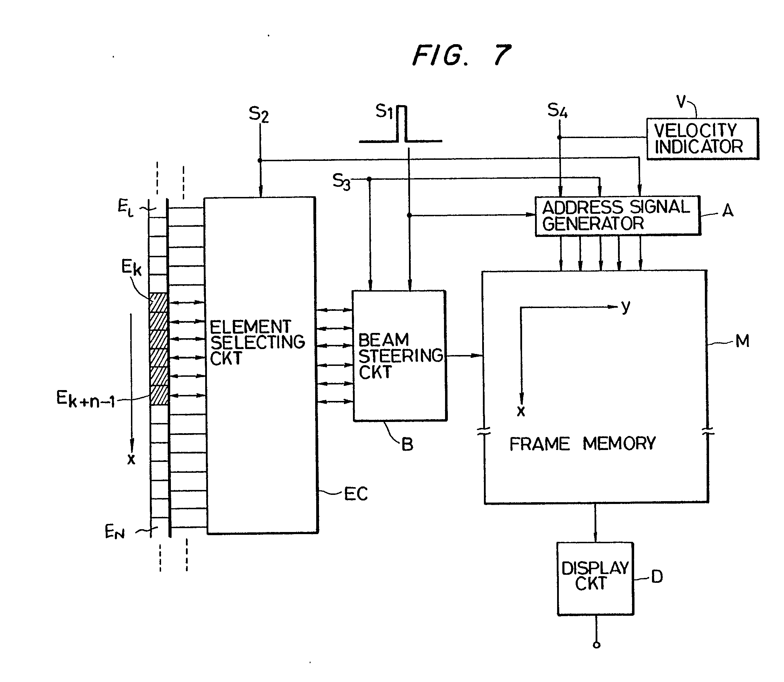

A fourth embodiment, shown in Figure 11(a), makes it possible to set the assumed acoustic velocity in the number k, i.e., from $S_{41}$ to $S_{4k}$. It is known that the acoustic velocity inside the body of the subject is not constant but varies by several percent from one part to another. Therefore, in the system of this fourth embodiment, k different acoustic velocities are assumed in the direction of depth during photography. The reception circuit R is constructed so that dynamic focus reception is achieved while changing the assumed acoustic velocity in k different ways in accordance with the passage of time from the transmission timing pulse.

Figure 11(b) shows the relation beteen the delay time $\tau$ and T in this case. The assumed acoustic velocity is changed step-wise in accordance with the passage of time T.

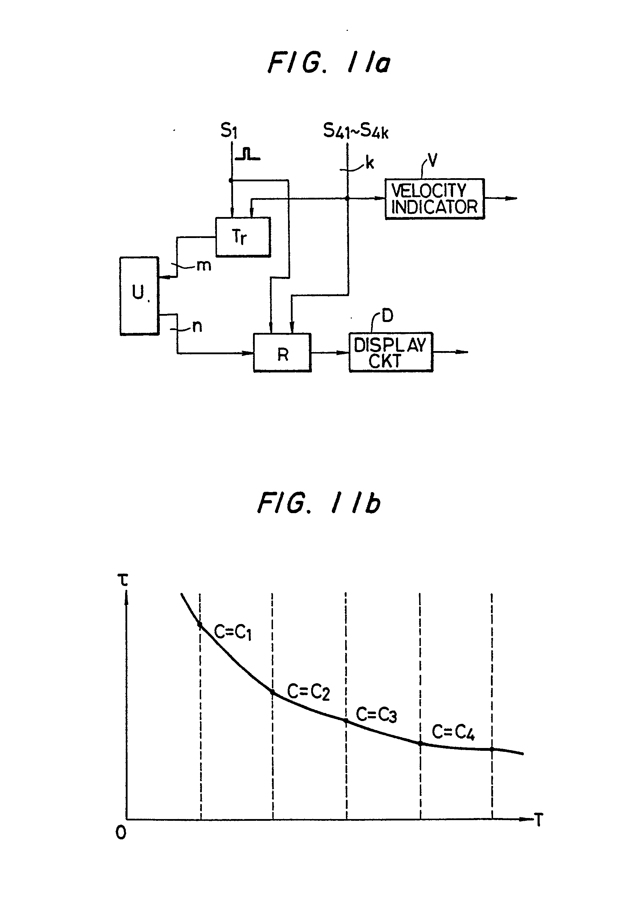

Figure 12 is a circuit diagram showing a fifth embodiment of the present invention. The circuit is similar to the second embodiment shown in Figure 9, except that the summation circuit for focusing is provided with the real time dynamic focus arrangements described above so as to take a photograph while changing the delay time in summation in accordance with the passage of time from the transmission timing pulse $S_1$ and with the assume acoustic velocity $S_4$. This arrangement makes it possible to take one photograph per transmission of the entire region expanding in the direction of depth in which a transmitted wave travels.

In the same way as in the system shown in Figure 7, the system of this fifth embodiment does not have any specific focal length and the reflection echo signal attains the state shown in Figure 6(B) for the reflector at all the distances when the acoustic velocity becomes $C_o$.

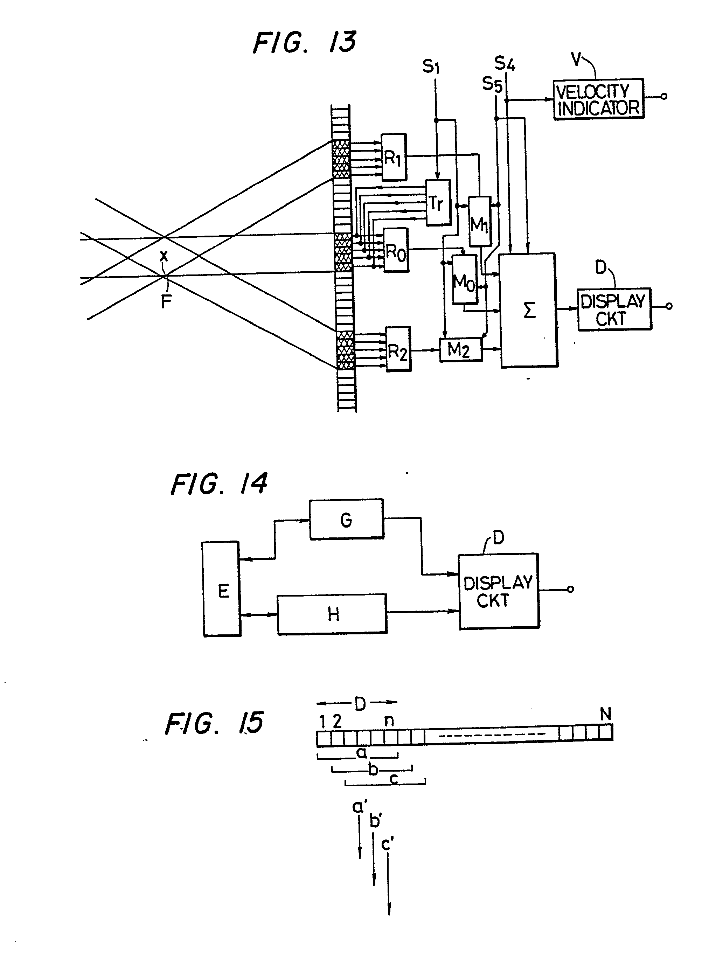

Figure 13 shows a sixth embodiment of the present invention. The difference between this embodiment and the fifth embodiment shown in Figure 12 is that the sixth embodiment has memories $M_o$, $M_1$ and $M_2$ that start writing by the transmission timing pulse $S_1$ and reading by a signal $S_5$ which is applied to the summation circuit $\Sigma$ for focusing in place of the transmission timing pulse $S_1$. This sixth embodiment has the advantage that since the reception signals are stored in the memories $M_o$, $M_1$, $M_2$, the assumed acoustic velocity signal $S_4$ for the same reception signal can be changed to display the image. This arrangement provides the effect that the determination of that value of the acoustic velocity which seems most reliable becomes easier.

Figure 14 shows an example of one application of the present invention. The signals received and to be transmitted by an oscillator array system E are alternately treated by both of a conventional ultrasonic imaging system G and an ultrasonic image imaging system H in accordance with the present invention. The output signals of both imaging systems G, H are transmitted to an image display circuit D and the two images are displayed either alternately or simultaneously. Since the image obtained by the ultrasonic photography system H is principally based upon the measurement of acoustic velocity, a system may be achieved which is easier to operate because it covers the problem that a side lobe level or the like becomes greater than that of the image formed by the conventional ultrasonic photography system G.

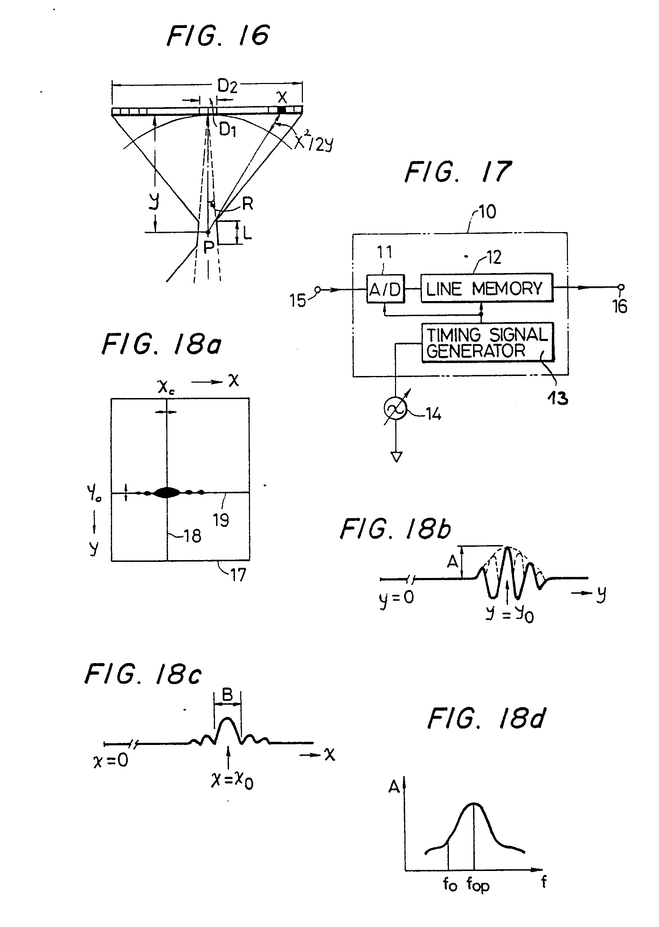

Next, a seventh embodiment of the present invention will be described. The principle of this embodiment will be discussed with reference to Figures 15 and 16 prior to the description of the seventh embodiment itself.

Figure 15 shows a conventional linear type ultrasonic diagnosis system. The elements of the probes are shown by symbols 1 to N and the elements arranged inside the transmission-reception diameter D are shown at 1 to n. When the position of the transmission-reception diameter is moved sequentially to a, b and c, the ultrasonic beam is also moved to a', b' and c'. In the conventional system, the transmission reception diameter is substantially the same and a relatively small diameter is employed to give an effective cost performance.

Figure 16 shows the principle of the seventh embodiment of the present invention, in which the transmission diameter $D_1$ is different from the reception diameter $D_2$ and this reception diameter $D_2$ is greater than that of the conventional system. Thus the direction characteristics of the transmission wave are determined substantially by the reception diameter $D_2$, with the resolution R and the depth of focus L being given by the following equation:

$$R = \lambda/D_2 \tag{11}$$

$$L = 4\lambda(y/D_2)^2 \tag{12}$$

where $\lambda$ is the wavelength and y is the depth.

When depth $y=100$ mm, wavelength $\lambda=0.43$ mm and the reception diameter $D_2=64$ mm, for example, the resolution $R=0.007$ (rad)$=0.4$ (deg.) and the depth of focus $L=4$ mm.

When the resolution is high and the depth of focus is small as described above, the influence of the acoustic velocity in a medium is large. In other words, high resolution cannot be obtained if the set acoustic velocity when designing the system deviates greatly from the acoustic velocity in the medium of the subject.

As shown in Figure 16, the following relation holds:

$$V_o \cdot \tau_o(x) = \sqrt{x^2+y^2} - y \tag{13}$$

where y is depth, x is the position of the selected element (the x origin being at the centre of the diameter) and $\tau_o(x)$ and $V_o$ are the initial values of the delay time for convergence and the acoustic velocity inside the medium, respectively. The right-hand side of equation (13) is determined solely by the geometric shape.

Assume that V is the true acoustic velocity inside the medium and the delay time $\tau_o(x)$ is changed to the delay time $\tau(x)$ so that a focussed image of a reflector at depth x is obtained. Then the following equation holds:

$$V \cdot \tau(x) = \sqrt{x^2+y^2} - y \tag{14}$$

From equation (13) and (14), the true acoustic velocity V in the medium is given by:

$$V = V_o \cdot \tau_o(x)/\tau(x) \tag{15}$$

Since $V_o$ and $\tau_o(x)$ are known, the acoustic velocity V can be measured by determining $\tau(x)$.

Figure 17 shows an embodiment of a one-channel reception wave delay circuit. The delay circuit comprises a delay circuit 10, an A—D convertor 11, a line memory 12, a timing signal generator 13, and a clock generator 14. The delay circuit has an input terminal 15 and an output terminal 16. When the clock frequency of the clock generator 14 is $f_o$, the delay time $\tau(x)$ when the clock frequency is changed to $f_{op}$ is given by the following equation, with $\tau_o$ (x) representing the

delay time of the delay circuit 10 for $f_o$:

$$\tau(x) = \tau_o(x) \cdot \frac{f_o}{f_{op}} \qquad (16)$$

From equation (15) and (16) therefore,

$$V = V_o \cdot \frac{f_{op}}{f_o} \qquad (17)$$

Therefore, the true acoustic velocity inside the medium can be measured from the change of the clock frequency $f_{op}/f_o$ of the variable delay means and the initial acoustic velocity $V_o$. At the same time, the depth y is also determined accurately.

It is theoretically possible for the operator to determine whether or not the reflector P is focused by observation but this method involves the problem that it is somewhat subjective. Hence, the embodiment now to be described is equipped with automatic focus means for adjusting the focus upon a predetermined reflector automatically.

Figures 18a to 18d illustrate the automatic focus system of this embodiment. Figure 18a is a schematic view of the point reflector displayed on a display 17 of an ultrasonic photography system and its position is $(x_o, y_o)$. However, $y_o$ does not always represent the actual distance to the point reflector and is a value obtained by halving the product of the assumed acoustic velocity $V_o$ and the time from the transmission. Here, y represents the direction of depth and x the lateral direction. This embodiment includes an x direction carcel 18 for positioning the point reflector to be focused in the display image in the x direction and a y direction carcel 19 for positioning it in the y direction and determining the initial value $f_o$ of the clock frequency corresponding to the depth.

In Figure 18b, an A mode waveform at $x=x_o$ is represented by a solid line and a detected waveform, by a dotted line.

The peak value of this detected waveform is assumed to be A.

Figure 18c shows the amplitude of the detected signals at a constant depth $y=y_o$ and scanned in the x direction. This is a direction beam pattern of the system and the beam width at the first zero point is assumed to be B.

Figure 18d shows the relationship between the frequency f of the clock generator 14 shown in Figure 17 and the detected waveform A in Figure 18b. In this drawing, the peak value A is maximum at $f_{op}$ which is different from the initial value $f_o$ of the clock frequency and a point target image is focused.

Similarly, it is obvious that the clock frequency f may be made variable so that the direction resolution B is minimum.

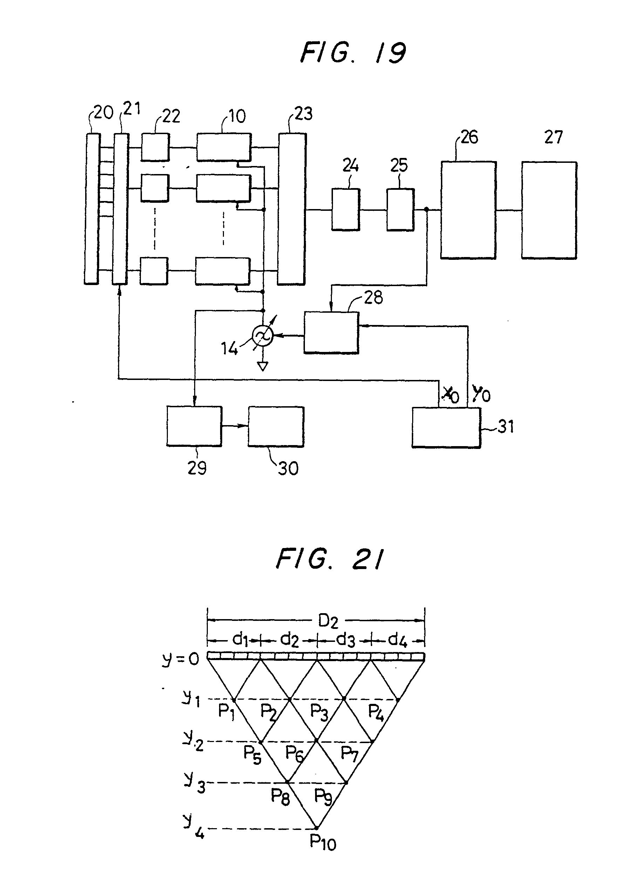

Figure 19 shows an example of the automatic focus system explained with reference to Figure 18. The system comprises oscillators 20, a selector 21 for selecting the transmission-reception diameter from all the elements arranged, a pre-amplifier 22, an adder 23, a compression circuit 24, a detection circuit 25; an image memory 26, an image display 27, and an automatic focus detection circuit 28 which detects the focal point by e.g. "mountain climbing" techniques. A calculation circuit 29 calculates the acoustic velocity from the change of the clock frequency in accordance with equation (17) and an operation panel 31 positions the carcels 18, 19 shown in Figure 18a to the position of the point target on the image and produces a position signal $x_o$, $y_o$ at that time.

In accordance with the construction described above, the carcel position $x_o$ is applied to the selector 21 to select the transmission-reception diameter. The reception signal is subjected to phase matching through the pre-amplifier 22, the delay means 10 and the adder 23, and the detected waveform shown in Figure 18b is obtained through the compression circuit 24 and the detection circuit 25. This detected waveform is applied to the automatic focus detection circuit 28.

A signal in the proximity of the depth $y=y_o$ is removed from the detected waveform for one line in the scanning direction $x=x_o$ in the automatic focus circuit 28, and the clock frequency f of the clock signal generator 14 is gradually increased from the initial value $f_o$ as shown in Figure 18d so as to detect automatically the clock frequency $f_{op}$ at which the peak value A of the detected waveform is maximum.

When $f_{op}$ has been determined as described above, the calculation circuit 29 performs the calculation of equation (17) to display the acoustic velocity V on a velocity indicator 30.

In the description above, no explanation has been given of the operation of the image memory 26. When the beam width of the beam pattern shown in Figure 18c is used as a parameter, however, it is necessary to change the clock frequency using one line data of the image memory 26 at the depth $y=y_o$ so that the beam width B is minimum.

In the embodiments described above, the mean acoustic velocity between the oscillator and the reflector is measured.

When a living body is to be dealt with, however, the medium acoustic velocity is generally nonuniform so that it is necessary to measure the acoustic velocity at each portion of the living body.

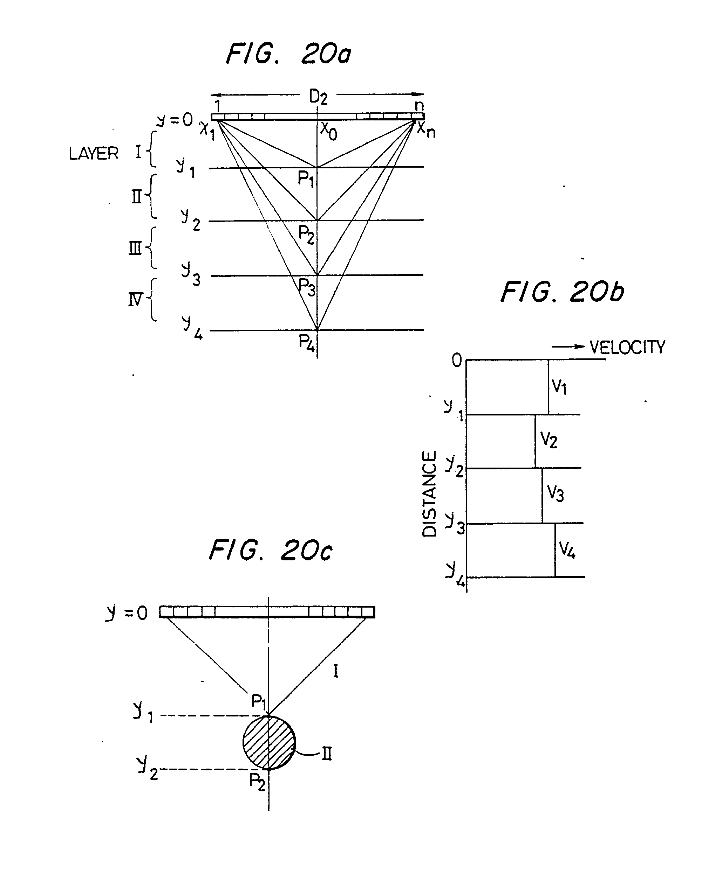

Therefore, an embodiment of the present invention will now be discussed, with reference to Figure 20, in which a medium having a nonuniform acoustic velocity is represented by a model with a structure layered in the direction of depth.

Assume that reflectors $P_1$, $P_2$ have depths $y_1$, $y_2$, respectively, the part between the oscillation and the reflector $P_1$ and the reflector $P_2$ consist of homogeneous media, which will be referred to as layers I and II, respectively.

The acoustic velocity $V_1$ of layer I is determined using a method of the present invention described already, and the position $y_1$, of the reflector $P_1$ is then determined in accordance with the following equation from the return time $T_1$ of the reflection wave:

$$y_1=T_1V_2/2 \qquad (18)$$

Next, the position $x_2$ of the reflector $P_2$ from the oscillator is likewise determined in accordance with the following equation:

$$y_2=T_2\hat{V}/2 \qquad (19)$$

where $\hat{V}$ is the mean acoustic velocity of the layers I and II.

The propagation time $T_3$ for going and returning inside layer II is given by

$$T_3=T_2-T_1 \qquad (20)$$

Thus, the acoustic velocity $V_2$ to be measured is given by:

$$V_2=\frac{2(y_2-y_1)}{T_3} \qquad (21)$$

When the subject has a multi-layerer structure consisting of layers I, II, III and IV such as shown in Figure 20a, this embodiment makes it possible to determine sequentially the acoustic velocities $V_1$, $V_2$, $V_3$, $V_4$ from the near distance side as shown in Figure 20b.

Even when a spherical heterogeneous substance such as a medium (II) exists in the homogeneous medium (I) such as shown in Figure 20d, this method makes it possible to measure the acoustic velocity inside the heterogeneous medium by setting the reflectors $P_1$ and $P_2$ as shown in the Figure.

In Figure 20a, the acoustic velocity is assumed to be uniform inside each of layers I to IV of the heterogeneous medium but in the case of a living body, it sometimes happens that the acoustic velocity is not uniform inside each layer in the x direction.

Figure 21 shows an embodiment of the present invention to measure the acoustic velocity at each part. The figure shows partial diameters $d_1$ to $d_4$ and reflectors $p_1$ to $p_{10}$. Mutually independent variable delay means are connected to the partial diameters $d_1$ to $d_4$, respectively.

First, the acoustic velocity between the depths $y=0$ and $y=y_1$ is measured for the reflectors $p_1$ to $p_4$ at a depth $y_1$ using the partial diameters $d_1$ to $d_4$. Next, the acoustic velocity between the depths $y=y_1$ and $y=y_2$ is measured for the reflectors $p_5$ to $p_7$ at the depth $y_2$ using the partial diameters $d_1+d_2$, $d_2+d_3$, and $d_3+d_4$, respectively. In the same way as described above, the acoustic velocity is measured sequentially for each of the reflectors $p_8$ to $p_{10}$.

In this manner, the acoustic velocity in each part can be measured sequentially for a medium which is heterogeneous not only in the depth direction but also in the lateral direction.

As described above, the present invention can automatically measure the mean acoustic velocity in a medium in accordance with the reflection method.

The present invention can also measure sequentially the acoustic velocity at each portion from a near distance side even when a medium is heterogeneous in the direction of depth or in tha lateral direction. The acoustic velocity distribution inside a body can also be measured.

The acoustic velocity data of the tissues of a living body thus obtained are useful for tissue characterisation and a significant contribution may be made to medical science.

**Claims**

1. An ultrasonic diagnosis system comprising:
an array (E; U) having a plurality of oscillation elements ($E_k-E_{k+n-1}$) for transmitting and receiving ultrasonic waves;
means (B, EC; $T_r$) for driving said oscillation elements so that pulse-like ultrasonic waves are transmitted from said array to an object;
means (A, B, D, EC, M; R; $R_o$, $R_1$, $R_2$) for detecting signals received by said oscillation elements, said signals being indicative of waves reflected from said object;
focusing means ($\Sigma$; 10, 23) for delaying received signals at different oscillation elements and for summing the delayed signals, said signals being delayed according to a range of delay times determined so that a received ultrasonic beam is focused to a focal point;
display means (D; 27) for displaying an image of said object by converting an output of said focusing means to a visual signal:
characterised in that said ultrasonic diagnosis system further comprises;
signal generating means for generating an adjustable signal ($S_4$) representative of an assumed acoustic velocity for said object, and indicating means (V: 30) for indicating said assumed velocity;
and in that said focusing means ($\Sigma$; 10, 23) is arranged to control said delay times as a function of said adjustable signal, whereby an actual acoustic velocity of said object is obtained from the indication of said indicating means (V; 30) when said signal generating means is adjusted so that the displayed image of a reflector in the object is in focus.

2. An ultrasonic diagnosis system according to claim 1, wherein said signal generating means is arranged to change sequentially the value of the assumed acoustic velocity from an initial value.

3. An ultrasonic diagnosis system according to claim 1 or claim 2, further comprising a detecting means (28) for detecting that the image of a reflector is in focus and for stopping the sequential change of said signal generating means upon detection of focus.

4. An ultrasonic diagnosis system according to claim 3, wherein said detecting means is arranged to detect that the image of a reflector is in focus through peak detection of the amplitude of said received signal.

5. An ultrasonic diagnosis system according to any one of the preceding claims, wherein the array is formed integrally as an acoustic wave transducer array.

6. An ultrasonic diagnosis system according to claim 5, wherein a greater number of elements of the transducer array are selected during reception and a smaller number of elements are selected during transmission so that the diameter used is changed between transmission and reception.

7. An ultrasonic diagnosis system according to claim 5 or claim 6, wherein three groups of elements forming the transducer array are selected.

8. An ultrasonic diagnosis system according to claim 7, wherein the three groups of elements are respectively at the center and each end of the transducer array.

**Patentansprüche**

1. Ultraschall-Diagnosesystem, aufweisend:
ein Array (E; U) mit einer Vielzahl von Schwingungselementen ($E_k - E_{k+n-1}$) zum Senden und Empfangen von Ultraschallwellen;
eine Einrichtung (B, EC; Tr) zum Ansteuern der Schwingungselemente, so daß impulsähnliche Ultraschallwellen von dem Array zu seinem Objekt übertragen werden;
eine Einrichtung (A, B, D, EC, M; R; R0, R1, R2) zum Erfassen von von den Schwingungselementen empfangenen Signalen, wobei diese Signale für von dem Objekt reflektierte Wellen kennzeichnend sind;
eine Fokussiereinrichtung ($\Sigma$; 10, 23) zum Verzögern von empfangenen Signalen an verschiedenen Schwingungselement und zum Summieren der verzögerten Signale, wobei diese Signale entsprechend einem Bereich von Verzögerungszeiten verzögert werden, der so vorgegeben ist, daß ein empfangener Ultraschallstrahl auf einen Brennpunkt fokussiert wird;
eine Sichtanzeigeeinrichtung (D; 27) zum Darstellen eines Bildes des Objekts durch Umwandeln einer Ausgabe der Fokussiereinrichtung in ein visuelles Signal;
dadurch gekennzeichnet,
daß das Ultraschall-Diagnosesystem weiterhin aufweist:
eine Signalerzeugungseinrichtung zum Erzeugen eines einstellbaren Signals ($S_4$), das für eine angenommene Schallgeschwindigkeit für das Objekt repräsentative ist, und
eine Indikatoreinrichtung (V; 30) zum Anzeigen der angenommenen Geschwindigkeit; und
daß die Fokussiereinrichtung ($\Sigma$; 10, 23) ausgelegt ist, die genannten Verzögerungszeiten als eine Funktion des einstellbaren Signals zu steuern, wodurch man ein tatsächliche Schallgeschwindigkeit des Objekts von der Anzeige der Indikatoreinrichtung (V; 30) erhält, wenn die Signalerzeugungseinrichtung so eingestellt wird, daß sich das dargestellte Bild eines Reflektors in dem Objekt im Brennpunkt befindet.

2. Ultraschall-Diagnosesystem nach Anspruch 1, wobei die Signalerzeugungeinrichtung ausgelegt ist, sequentiell den Wert der angenommenen Schallgeschwindigkeit ausgehend von einem Anfangswert zu verändern.

3. Ultraschall-Diagnosesystem nach Anspruch 1 oder Anspruch 2, weiterhin aufweisend eine Erfassungseinrichtung (28) zum Erfassen, daß sich das Bild eines Reflektors im Brennpunkt befindet, und zum Anhalten der sequentiellen Veränderung der Signalerzeugungseinrichtung bei Brennpunkt-Erfassung.

4. Ultraschall-Diagnosesystem nach Anspruch 3, wobei die Efassungseinrichtung ausgelegt ist, durch Spitzenwert-Erfassung der Amplitude des empfangenen Signals zu erfassen, daß sich das Bild eines Reflzktors im Brennpunkt befindet.

5. Ultraschall-Diagnosesystem nach einem der vorhergemsnden Ansprüche, wobei das Array integral als ein Schalellen-Wandlerarray ausgebildet ist.

6. Ultraschall-Diagnosesystem nach Anspruch 5, wobei beim Empfangen eine größere Anzahl von Elementen des Wandlerarray und beim Senden eine kleinere Anzahl von Elementen ausgewählt wird, so daß der verwendete Durchmesser zwischen Senden und Empfangen verändert wird.

7. Ultraschall-Diagnosesystem nach Anspruch 5 oder Anspruch 6, wobei drei Gruppen von Elementen, die das Wandlerarray bilden, gewählt werden.

8. Ultraschall-Diagnosesystem nach Anspruch 7, wobei sich die drei Gruppen von Elementen im Zentrum bzw. an jedem Ende des Wandlerarray befinden.

**Revendications**

1. Système de diagnostic par ultrasons comprenant
un réseau (E; U) possédant une pluralité d'éléments oscillants ($E_k - E_{k+n-1}$) servant à émettre et recevoir des ondes ultrasonores; des moyens (B, EC; Tr) servant à commander lesdits éléments oscillants de manière que des ondes ultrasonores en forme d'impulsions soient émises depuis ledit réseau en direction d'un objet;
des moyens (A, B, D, EC, M; R; $R_0$, $R_1$, $R_2$) servant à détecter des signaux reçus par lesdits éléments oscillants, indicatifs d'ondes réfléchies par ledit objet;
des moyens de focalisation ($\Sigma$; 10, 23) servant à retarder les signaux reçus au niveau de différents éléments oscillants et à réaliser la sommation des signaux retardés, lesdits signaux étant retardés conformément à une gamme de retards déterminés de sorte qu'un faisceau ultrasonore reçu est focalisé en un foyer;
des moyens d'affichage (D; 27) servant à afficher une image dudit objet par transformation d'un signal de sortie desdits moyens de focalisation en un signal optique;

caractérisé en ce que ledit système de diagnostic ultrasonore comporte en outre:

des moyens de production de signal servant à produire un signal réglable ($S_4$) représentatif d'une vitesse acoustique supposée pour ledit objet, et

des moyens indicateurs (V; 30) servant à indiquer ladite vitesse supposée;

et en ce que les moyens de focalisation ($\Sigma$; 10, 23) sont disposés de manière à commander lesdits retards en fonction desdits signaux réglables, ce qui a pour effet qu'une vitesse acoustique réelle dudit objet est obtenue à partir de l'indication fournie par lesdits moyens indicateurs (V; 30) lorsque lesdits moyens de production de signal sont réglés de manière que l'image affichée d'un réflecteur situé sur l'objet soit focalisée.

2. Système de diagnostic par ultrasons selon la revendication 1, dans lequel lesdits moyens de production de signal sont agencés de manière à modifier de façon séquentielle la valeur de la vitesse acoustique supposée à partir d'une valeur initiale.

3. Système de diagnostic par ultrasons selon la revendication 1 ou 2, comprenant en outre des moyens de détection (28) servant à détecter fait que l'image d'un réflecteur est focalisée, et arrêter la modification séquentielle desdits moyens de production de signal lors de la détection de l'état focalisé.

4. Système de diagnostic à ultrasons selon la revendication 3, dans lequel lesdits moyens de détection sont agencés de manière à détecter le fait que l'image d'un réfleteur est focalisée, grâce à la detection du maximum de l'amplitude dudit signal reçu.

5. Système de diagnostic par ultrasons selon l'une quelconque des revendications précédentes, dans lequel le réseau est formé d'un seul tenant sous la forme d'un réseau de transducteurs d'ondes acoustiques.

6. Système de diagnostic par ultrasons selon la revendication 5, dans lequel un nombre plus important d'éléments du réseau de transducteurs est sélectionné pendant la réception et un nombre plus faible d'éléments est sélectionné pendant l'émission de sorte que le diamètre utilisé est changé entre l'émission et la réception.

7. Système de diagnostic par ultrasons selon la revendication 5 ou 6, dans lequel trois groupes d'éléments constituant le réseau de transducteurs sont sélectionnés.

8. Système de diagnostic par ultrasons selon la revendication 7, dans lequel les trois groupes d'éléments sont situés respectivement au centre et à chaque extrémité du réseau de transducteurs.

## FIG. 1

## FIG. 2

*FIG. 3*

$$D$$

$$0$$

$$x$$

$$F_0 \quad Y_0 = C_0 T_0$$

$$C_0$$

$$y$$

*FIG. 4*

$$0 \qquad\qquad x$$

$$P$$

$$y$$

*FIG. 5*

$$D$$

$$E_1 \qquad E_2 \qquad E_3$$

$$x$$

$$Y$$

$$F$$

2

## FIG. 6

(A) $CY > C_0 Y_0$

(B) $CY = C_0 Y_0$

(C) $CY < C_0 Y_0$

## FIG. 8

# FIG. 9

Ej
Ej+n-1
Ek
Ek+n-1
Eℓ
Eℓ+n-1
x
F
S1
S4
V
VELOCITY INDICATOR
R1
Tr
R0
R2
Σ
D
DISPLAY CKT

# FIG. 12

x
F
S1
S4
V
VELOCITY INDICATOR
R1
Tr
R0
R2
Σ
D
DISPLAY CKT

# FIG. 10a

# FIG. 10b

$$\tau(T) = \sqrt{\left(\frac{X}{C}\right)^2 + \left(\frac{T}{2}\right)^2} - \frac{T}{2}$$

FIG. 7

## FIG. IIa

## FIG. IIb

7

FIG. 13

VELOCITY INDICATOR

DISPLAY CKT

FIG. 14

DISPLAY CKT

FIG. 15

*FIG. 16*

*FIG. 17*

*FIG. 18a*

*FIG. 18b*

*FIG. 18c*

*FIG. 18d*

# FIG. 19

# FIG. 21

# FIG. 20a

# FIG. 20b

# FIG. 20c